# EUROPEAN PATENT SPECIFICATION

(11) **EP 0 409 155 B1**
(45) Date of publication and mention of the grant of the patent: **11.05.1994**
(21) Application number: 90113643.2
(22) Date of filing: 17.07.1990
(51) Int. Cl.: C08L 53/00, C08K 5/12, A61J 1/00

(54) **Bag for storage of platelets and multiple bag system containing the bag**
Beutel für die Lagerung von Blutplättchen und den Beutel enthaltendes Mehrfachbeutelsystem
Sac pour le magasinage de plaquettes sanguines et système de sacs multiples contenant ce sac

(30) Priority: 20.07.1989 JP 188515/89
(43) Date of publication of application: 23.01.1991
(73) Proprietor: NISSHO CORPORATION, Osaka-shi (JP)
(72) Inventor: Fukui, Kiyoshi, Uji-shi, Kyoto-fu (JP); Kawamoto, Shoji, Takatsuki-shi, Osaka-fu (JP); Futagawa, Hitoshi, Otsu-shi, Shiga-ken (JP)
(74) Representative: Türk, Gille, Hrabal, Leifert

(56) References cited:
- EP-A- 0 330 151
- US-A- 4 286 597

## Description

The present invention relates to a bag for storage of platelets and a multiple bag system containing the bag, and more particularly to a bag for storage of platelets and a multiple bag system in which the blood platelet can be stored for a long period of time since the blood platelet does not adhere onto the inner surface of the bags when the blood platelet is stored in the bags and the bags would not be deformed due to a procedure of separating platelets from whole blood.

As a material of a bag for storage of platelets which can be safely stored for a long period of time and can be supplied to a patient in case of necessity, there is proposed a polymer composition as a resin not containing a plasticizer, prepared by mixing (A) poly(ethylene-butylene)-polystyrene block copolymer with (B) polypropylene and (C) ethylene-vinyl acetate copolymer or polyethylene as disclosed in U.S. Pat. No. 4,140,162. It is disclosed in U.S. Pat. No. 4,222,379 that the above resin is used as a material of a transfer bag used in a multiple bag system comprising a donor bag for receiving and storing blood from a blood donor and at least one transfer bag for storing blood platelet separated from the blood plasma which is gotten by centrifugating the blood in the donor bag.

The transfer bag used herein has an advantage that additives such as a plasticizer contained in the bag do not elute into the blood platelet because additives are not contained in the bag. However, the bag has disadvantages that the bag is transformed when the bag is subjected to high-pressure steam sterilization because elastic modulus of the material of the bag is lowered at high temperatures. Particularly, the bag made of a polymer mixture, in which ethylene-vinyl acetate copolymer is contained, has a defect that acetic acid, which is generated from the bag as the results of heat decomposition at the time of forming, or hydrolysis at the time of high-pressure steam sterilization, is dissolved into a concentrated solution of blood platelet so that the concentrated solution is acidified.

As a bag for storage of platelets from which the above-mentioned disadvantages are removed, a bag for storage of platelets made of polymer alloy prepared by mixing poly(ethylene-butylene)-polystyrene block copolymer and polypropylene with ethylene-acrylic acid ester copolymer has been proposed in EP-A-330151 (Art.54(3,4)EPC). And it has been found that no chemical substances elute from the bag into the blood platelet, that the bag is not deformed at high or low temperatures, and that the bag has high gas permeability, therefore the bag can be used as a bag for storage of platelets for a long period of time.

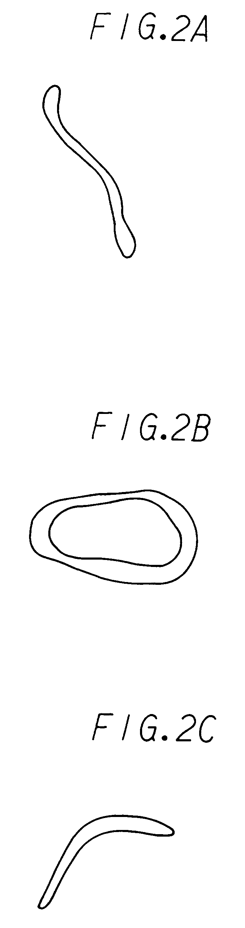

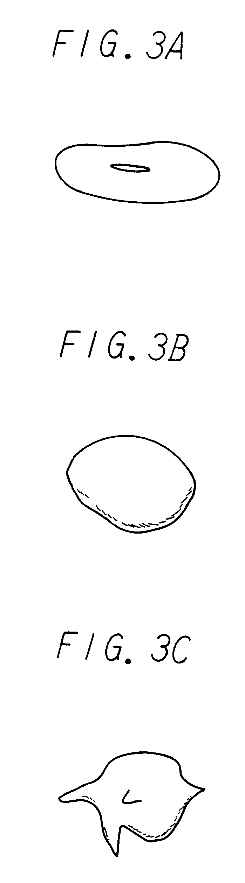

However, it has been found that when platelets are stored in the bag made of the polymer alloy, the shape of the platelets begins to change after 2 days of storage and the platelets show the appearance of unclassified shapes such as an elongated tubular shape, a doughnut shape or a crescent shape as shown in Figs. 2A to 2C when the platelets are observed using an optical microscope. The shape of the platelets stored in the bag made of the polymer alloy is apparently different from the shapes shown in Figs. 3A to 3C such as a discoid shape, a spherical shape and a discoid shape with small projections which are known as the usual shapes of platelets observed when the platelets are stored in the bag made of polyvinyl chloride. Therefore, it is thought that damage is done to the platelets stored in the bag made of the polymer alloy.

Further, the bag made of the polymer alloy has a defect that the bag is partly deformed and generates puckers, and the blood is remained in the puckers and therefore the strength of the deformed parts of the bag deteriorates at the time of separating the platelets from the blood plasma in the bag by means of intense centrifugation after the blood plasma is transferred from a donor bag and stored in the above bag made of the polymer alloy.

The present invention has been studied to give a bag for storage of platelets from which the above-mentioned disadvantages are removed. As the results of researches, when blood platelets are stored in a bag for storage of platelets made of a resin comprising the polymer alloy and a specific amount of plasticizer, it has been eventually found that the function of platelets is not lowered.

The present invention has been accomplished in accordance with the above-mentioned results.

These and other objects of the present invention will become apparent from the description hereinafter.

In accordance with the present invention, there is provided a bag for storage of platelets made of a resin comprising a polymer alloy consisting essentially of (a) 25 to 60 % by weight of poly(ethylene-butylene)polystyrene block copolymer, (b) 20 to 40 % by weight of polypropylene, and (c) 5 to 35 % by weight of ethylene-acrylic acid ester copolymer, the segment of the polystyrene in the molecular chains of the polymer (a) having at least 2 oriented portions and the molecular chains of the polymers (a), (b) and (c) being entangled to form a network structure, and 1 to 20 % by weight of a plasticizer.

In accordance with the present invention, there is also provided the bag for storage of platelets, wherein the plasticizer is at least one member selected from the group of dioctyl phthalate, dioctyl adipate, tri-n-octyl ester trimellitate and tri-2-ethylhexyl trimellitate.

In accordance with the present invention, there is also provided a multiple bag system comprising (a) a donor bag for receiving blood from a blood donor and separating the components of the blood, (b) one or more than one transfer bag for storing the separated blood components, and (c) tubes for connecting a bag with the other bag and transferring the separated blood components, wherein at least one transfer bag is the above-mentioned bag for storage of platelets.

Fig. 1 is a schematic explanatory drawing showing a multiple bag system of the present invention.

Figs. 2A to 2C are schematic drawings showing appearances of blood platelets having an unclassified shape observed by means of an optical microscope.

Figs. 3A to 3C are schematic drawings showing usual appearances of blood platelets observed by means of an optical microscope.

In the present invention, a polymer alloy is prepared by mixing ethylene-acrylic acid ester copolymer with a mixture of poly(ethylene-butylene)-polystyrene block copolymer and polypropylene in a specific ratio, because the ethylene-acrylic acid ester copolymer acts as an agent which gives compatibility to the poly(ethylene-butylene)-polystyrene block copolymer and polypropylene. When a bag made of a resin prepared by mixing the polymer alloy with a specific amount of a plasticizer is used as a bag for storage of platelets, it is thought that the bag posseses a function for storing platelets because the plasticizer uniformly mixed with the polymer alloy is eluted into the platelets from the inner surface of the bag in a small amount or a significant amount and the plasticizer prevents the shape of platelets from changing into an unclassified shape.

A poly(ethylene-butylene)-polystyrene block copolymer used in the present invention comprises a hard segment and a soft segment. The hard segment is composed of a styrene polymer such as polystyrene or polymethylstyrene, and the soft segment is composed of a polyethylene-butylene copolymer. The polystyrene polymer used as a hard segment has a number average molecular weight of 5,000 to 120,000. The polyethylene-butylene copolymer used as a soft segment can be prepared by reacting polybutadiene having a number average molecular weight of 10,000 to 250,000 with polystyrene to give a poly(ethylene-butadiene)-polystyrene block copolymer, and thereafter hydrogenerating the block copolymer. The preferable molar ratio of ethylene and butylene contained in the polyethylene-butylene copolymer, which is a soft segment, is 40/60 to 60/40, and the preferable content of the soft segment in the block copolymer is 60 to 90 % by weight in order to give a block copolymer having excellent mechanical property and flexibility. The content of the block copolymer in the polymer mixture is 25 to 60 % by weight, preferably 30 to 50 % by weight. When the content is less than 25 % by weight, there is a tendency that the flexibility of an obtained bag deteriorates, and when the content is more than 60 % by weight, there is a tendency that the moldability of the obtained bag deteriorates.

It is preferable that the number average molecular weight of the block copolymer is about 15,000 to 370,000, more preferably 30,000 to 250,000.

The terminology "polypropylene" used in the present invention is intended to refer to a homopolymer of propylene or a copolymer of which main component is propylene. Examples of the component other than propylene used in the copolymer are, for instance, ethylene, and 1-butene. The content of the component other than propylene is preferably at least 10 % by weight. The polypropylene has an ability to give a bag mechanical strength at the time of forming, and the mechanical strength of the bag is remarkably improved by mixing the polypropylene with the other polymer in a specific ratio when preparing a polymer alloy. The content of the polypropylene in the polymer mixture is 20 to 40 % by weight, preferably 25 to 35 % by weight. When the content is less than 20 % by weight, there is a tendency that the transparency and mechanical strength of an obtained bag deteriorate, and when the content is more than 40 % by weight, there is the tendency that the flexibility of the obtained bag deteriorates.

The ethylene-acrylic acid ester copolymer gives the block copolymer and polypropylene compatibility and contributes to the formation of a polymer alloy. Examples of the acrylic acid ester used in the present invention are, for instance, methyl acrylate, ethyl acrylate, and butyl acrylate. The content of the acrylic acid ester in the copolymer is preferably 5 to 90 % by mole. When the content of acrylic acid ester is less than 5 % by mole, there is a tendency that transparency of the ethylene-acrylic acid ester copolymer deteriorates, and when the content of the acrylic acid ester is more than 90 % by mole, there is a tendency that the elastic modulus of the ethylene-acrylic acid ester copolymer at high temperatures deteriorates.

The content of the ethylene-acrylic acid ester copolymer in the polymer mixture is 5 to 35 % by weight, preferably 10 to 30 % by weight. When the content is less than 5 % by weight, there is a tendency that the dispersibility of the ethylene-acrylic acid ester copolymer in the polymer mixture and the workability at extruding deteriorate and the transparency of the polymer mixture is lowered. Further, there is a tendency that the physical properties at low temperatures deteriorate. When the content is more than 35 % by weight, there is a tendency that the mechanical strength of an obtained bag is lowered.

When the homogeneously mixed polymer mixture is subjected to the melt extrusion molding, the molecular chains of polystyrene segments which are hard segments of the block copolymer gather to form at least two oriented portions in the polymer mixture, and the polymer mixture forms a polymer alloy having a chemical constitution that the chains of a soft segment of the block copolymer, a hard segment of the block copolymer which is not oriented, polypropylene and ethylene-acrylic acid ester copolymer are entangled together to form a network structure.

The polymer mixture is formed into a polymer alloy having preferable properties required for a bag for storage of platelets, such as mechanical strength, in particular mechanical property at low temperatures and improved gas permeability such as oxygen permeability or carbon dioxide gas permeability.

In the present invention, a plasticizer is added to the polymer alloy. It is preferable that the plasticizer is at least one member selected from the group consisting essentially of dioctyl phthalate, dioctyl adipate, tri-n-octyl trimellitate and tri-2-ethylhexyl trimellitate. These plasticizers can be uniformly mixed with the polymer alloy. The amount of the plasticizer added to the polymer alloy is 1 to 20 % by weight, preferably 2 to 10 % by weight on the basis of the polymer alloy. When the amount is less than 1 % by weight, there are tendencies that platelets having an unclassified shape increase during the platelets are stored in the obtained bag, and that the bag is partly yielded at the time of separating platelets from blood plasma by means of an intense centrifugation. When the amount is more than 20 % by weight, there is a tendency that the amount of the plasticizer eluted from inner surface of the bag is so large that the function of the platelets is lowered.

After the above-mentioned polymer alloy and plasticizer are homogenously mixed together in a blender or a mixer, the mixture is melted. The melted mixture is extruded into a mold having a shape corresponding to a medical bag and is thereafter subjected to blow molding, or is extruded to give a sheet and is subsequently heat-sealed at the outer side of the sheet to give a bag having a prescribed shape. The thickness of the sheet can be suitably adjusted in accordance with the uses. The thickness is, for instance, 200 to 450 µm.

A multiple bag system of the present invention comprises a donor bag, one or more than one transfer bags and tubes. The number of the transfer bags is one or more than one as mentioned above, but can be suitably adjusted to, for instance, 1 to 4 as occasion demands.

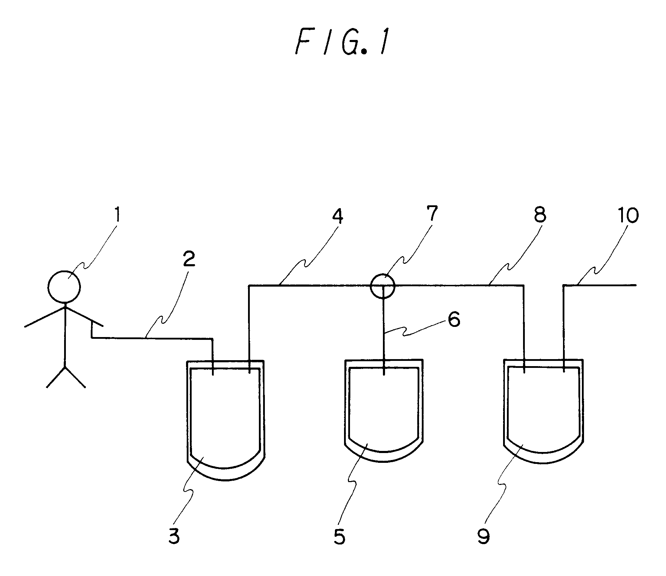

One embodiment of the bag for storage of platelets of the present invention, which is used as a transfer bag of a multiple bag system, is explained in accordance with Fig. 1.

Fig. 1 is a drawing explaining an embodiment of a multiple bag. In Fig. 1, 1 denotes a blood donor, each of 2, 4, 6, 8 and 10 denotes a tube, 3 denotes a donor bag, 5 denotes a first transfer bag, 7 denotes a directional control valve, and 9 denotes a second transfer bag.

Blood collected from the blood donor 1 is transferred into the donor bag 3. The blood is separated into blood corpuscle component and blood plasma component in the donor bag 3 by means of a centrifugal separator. It is preferable that a material of the donor bag 3 is a plastic material containing a plasticizer. Examples of the plastic material are, for instance, polyvinyl chloride, polyolefine, and the above-mentioned polymer alloy

The content of the plasticizer in the plastic material can be selectively adjusted in accordance with the kind of the plasticizer and the combination of the plasticizer and the plastic material. The content is, for instance, 1 to 50 % by weight.

The blood plasma component separated from the blood in the donor bag 3 is transferred into the first transfer bag 5 through the tube 4 and the tube 6. The tubes 4 and the tube 6 are, for instance, made of polyvinyl chloride, and polyolefine,.

The first transfer bag 5 is made of the above-mentioned polymer alloy containing the plasticizer. Also, the blood plasma components in the first transfer bag 5 are subjected to intense centrifugation and separated into blood platelet component and blood plasma component other than the blood platelet by means of a centrifugal separator, and the blood plasma component other than the blood platelet is transferred into the second transfer bag 9 through the tube 6 to the tube 8. The blood platelet is stored in the first transfer bag 5.

Examples of the material of the second transfer bag 9 are, for instance, the above-mentioned polymer alloy containing a plasticizer, polyvinyl chloride containing dioctyl phthalate, and polyvinyl chloride containing 2-ethylhexyl trimellitate. The blood plasma component other than blood platelet, which is accomodated in the second transfer bag 9, is usually stored in a freezer.

The bag for storage of platelet and the multiple bag system containing the bag of the present invention is more specifically described and explained by means of the following Examples and Comparative Examples. However, it is to be understood that the present invention is not limited to these Examples, and various changes and modifications can be made in the invention without departing from the spirit and scope thereof.

### Examples 1 to 4

A mixture composed of 40 % by weight of poly(ethylene-butylene)-polystyrene block copolymer (available from Shell Chemical Co., Ltd. under the trade name "KRATON G-1650"), 30 % by weight of polypropylene (available from Sumitomo Chemical Company, Limited under the trade name "FL-6711N") and 30 % by weight of ethylene-ethyl acrylate copolymer containing 15% by mole of ethyl acrylate (available from Japan Uniker Co., Ltd. under the trade name "DPDJ 6182") was prepared by mixing these components with a mixer. The mixture was heated to a temperature of 200°C to melt to give a polymer alloy. A mixture of a pellet made of the polymer alloy and dioctyl phthalate (hereinafter referred to as "DOP") was extruded to give a sheet having a thickness of 250 µm. The amount of the DOP is shown in Table 1. The sheet was set on a mold having a shape corresponding to a bag and cut with heating to give a sheet having a shape corresponding to a bag. Two sheets were overlapped and all peripheral portions of the overlapped two sheets other than one side thereof were heat-sealed to give a bag having one open end. After that, the open end of the bag was fitted with tubes, an inlet and a protector by melting and fusing the sheet to give a bag having a volume of 150 mℓ.

A donor bag made of polyvinyl chloride containing 35 % by weight of DOP was charged with 28 mℓ of CPD solution and 200 mℓ of whole blood collected from a man of health. The whole blood was separated into blood corpuscle component and blood plasma component in the donor bag by centrifugating the whole blood at 2000 rpm. The blood plasma component was transferred to the first transfer bag obtained in the present invention, then blood platelet was separated from the blood plasma by intensely centrifugating the first transfer bag charged with the blood plasma component at 3800 rpm. The blood plasma component other than the blood platelet was transferred to a second transfer bag made of the same resin as the first transfer bag. The blood platelet remained in the first transfer bag was intended to as one unit of blood platelet preparation. After a bag having a volume of 1.0 ℓ made of the above-mentioned polymer alloy was charged with 4 units of the same type of the blood platelet preparations obtained in Examples 1 to 4, 20 mℓ of the platelets was poured into the 4 kinds of the bag obtained in the Examples, respectively. Aggregation property of the blood platelet after the bag was subjected to vibration at 60 rpm at 22°C for 72 hours and deformation of the bag were investigated in accordance with the following methods. The results are shown in Table 1.

### (1) Aggregation property of the blood platelet

After 4 mM of CaCℓ₂ was added to the blood platelet, maximum aggregation percentage caused by adding 20 µm of adenosine diphosphate (hereinafter referred to as "ADP") was measured.

### (2) Deformation of the bag

A state of the surface of the first transfer bag charged with the blood plasma component at the time of subjected to intense centrifugation was evaluated in accordance with the following criteria.

### (Criteria)

- ○ :: No change generated on the surface.
- △ :: Bands having a convex shape generated on the bottom face of the bag.
- X :: Round convex portions generated on the bottom face of the bag.

### Comparative Example 1

The same procedure as in Example 1 was repeated except that the polymer alloy which does not contain DOP was used as a material of the first transfer bag.

Aggregation property of the blood platelet and deformation of the bag were investigated in the same manner as in Example 1. The results are shown in Table 1.

### Comparative Example 2

The same procedure as in Example 1 was repeated except that a bag made of polyvinyl chloride sheet containing 35 % by weight of dioctyl phthalate and having a thickness of 250 µm was used as the first transfer bag.

Aggregation property of the blood platelet and deformation of the bag were investigated in the same manner as in Example 1. The results are shown in Table 1.

**Table 1**

| Ex. No. | Amount of DOP (% by weight) | Amount of DOP eluted into blood platelet (ppm) | Aggregation property of blood platelet (%) | Deformation of the bag |
|---|---|---|---|---|
| 1 | 1 | 14.0 | 70 | ○ |
| 2 | 5 | 15.1 | 62 | ○ |
| 3 | 15 | 26.6 | 53 | ○ |
| 4 | 20 | 33.5 | 48 | △ |

| Com.Ex.No. | | | | |
|---|---|---|---|---|
| 1 | 0 | 3.2 | 77 | X |
| 2 | - | 469.9 | 18 | ○ |

As is clear from Table 1, the amount of DOP eluted into the blood platelet is small during the blood platelet is stored in the bags obtained in Examples 1 to 4 of the present invention, which are made of the polymer alloy containing 1 to 20 % by weight of DOP.

Also, the blood platelet stored in the bags of the present invention has aggregation property after 72 hours of storage.

In addition, the bag of the present invention is not deformed at the time of subjected to intense centrifugation.

### Examples 5 to 8

The same procedure as in Example 1 was repeated except that the material used in the first transfer bag was changed into the polymer alloy containing tri-2-ethylhexyl trimellitate (hereinafter referred to as "TOTM") in an amount shown in Table 2. Aggregation property of the blood platelet and deformation of the bag were investigated in the same manner as in Example 1. The results are shown in Table 2.

**Table 2**

| Ex. No. | Amount of TOTM (% by weight) | Amount of TOTM eluted into blood platelet (ppm) | Aggregation property of blood platelet (%) | Deformation of the bag |
|---|---|---|---|---|
| 5 | 1 | 0.15 | 74 | ○ |
| 6 | 5 | 0.21 | 69 | ○ |
| 7 | 15 | 0.30 | 62 | ○ |
| 8 | 20 | 0.38 | 55 | △ |

As is clear from Table 2, the amount of TOTM eluted into the blood platelet is small during the blood platelet is stored in the bags obtained in Examples 5 to 8 of the present invention which are made of the polymer alloy containing 1 to 20 % by weight of TOTM.

Also, the blood platelet stored in the bags of the present invention has aggregation property after 72 hours of storage.

In addition, the bag of the present invention is not deformed at the time of subjected to intense centrifugation.

### (Gas permeability test of bag)

With respect to the bags obtained in Examples 2 and 6 and Comparative Example 2, oxygen permeability, carbon dioxide gas permeability and steam permeability were measured in accordance with the following methods. The results are shown in Table 3.

### (1) Oxygen permeability

Oxygen permeability of the bag was measured in accordance with ASTM-D-1434.

### (2) Carbon dioxide gas permeability

Carbon dioxide gas permeability of the bag was measured in accordance with ASTM-D-1434.

### (3) Steam permeability

Steam permeability of the bag was measured in accordance with JIS-Z-0208.

**Table 3**

| Ex.No. | Oxygen permeability (cc/m²·atm·24hrs) | Carbon dioxide gas permeability (cc/m²·atm·24hrs) | Steam permeability (g/m²·24hrs) |
|---|---|---|---|
| 2 | 1920 | 6821 | 5.6 |
| 6 | 2033 | 7154 | 5.9 |

| Com.Ex.No. | | | |
|---|---|---|---|
| 2 | 580 | 2694 | 21.0 |

As is clear from Table 3, the bag of the present invention has oxygen permeability of about at least 3.3 times larger than that of the bag obtained in Comparative Example 2, and carbon dioxide gas permeability of about at least 2.5 times larger than that of the bag obtained in Comparative Example 2, and steam permeability of about at most 1/3.6 of the steam permeability of the bag obtained in Comparative Example 2. Accordingly, the bags obtained in Examples 2 and 6 can be preferably used as a bag for storage of platelets in comparison with the bag obtained in Comparative Example 2.

### Comparative Example 3

A bag having a volume of 150 mℓ was produced in the same manner as in Examples 1 to 4 except that ethylene-vinyl acetate copolymer containing 15 % by weight of vinyl acetate was used instead of ethylene-ethyl acrylate copolymer and that a plasticizer was not used.

The bag was charged with 20 mℓ of blood platelet. Ratio of generation of blood platelet having an unclassified shape in blood platelet stored in the bag after the storage of 3 days or 6 days was measured in accordance with the following methods. The results are shown in Table 4.

### (Ratio of generation of blood platelet having an unclassified shape)

With respect to the bags obtained in Example 2 and Comparative Examples 1 to 3, ratio of generation of blood platelet having an unclassified shape was measured as follows.

Concentrated blood platelet solution was fixed in glutaraldehyde solution and changes of the shape of blood platelet was observed using Nomarski interference-contrast light microscope B-22 (available from Ernst Leitz Wetzlar GmbH).

Ratio of blood platelet having an unclassified shape on the basis of all blood platelets was measured by counting the blood platelet by means of an electronic cell counter (available from Toa Medical Engineering, trade name: Sysmex E 500).

**Table 4**

| Ex.No. | Ratio of generation of blood platelet having an unclassified shape (%) | |
|---|---|---|
| | After storage of 3 days | After storage of 6 days |
| 2 | 2.3 | 4.2 |

| Com.Ex.No. | | |
|---|---|---|
| 1 | 2.9 | 6.1 |
| 2 | 2.1 | 4.0 |
| 3 | 5.2 | 9.8 |

As is clear from Table 4, the bag of the present invention obtained in Example 2 has lower ratio of blood platelet having an unclassified shape generated in the bag during the blood platelet is stored in the bag as well as the bag made of polyvinyl chloride obtained in Comparative Example 2 has.

On the contrary, the bag made of a polymer alloy which does not contain a plasticizer obtained in Comparative Example 1 and the bag made of polyolefine which does not contain a plasticizer obtained in Comparative Example 3 have large ratio of blood platelet having an unclassified shape generated in the bag during the blood platelet is stored in the bag.

As mentioned above, blood platelet can be stored in a bag made of the resin comprising the polymer alloy of the present invention safely for a long period of time because there are occurred little adherence and aggregation of the blood platelet on the inner surface of the bag, and the amount of blood platelet having an unclassified shape generated is small during the blood platelet is stored in the bag.

It can be said that the bag of the present invention can be preferably used as a bag for storage of platelets since the bag has preferable oxygen permeability and carbon dioxide gas permeability.

Also, surfaces of the bag for storage of platelets of the present invention are not deformed by high speed rotation at the time of separating blood platelet from blood plasma by means of intense centrifugation. Accordingly, the bag of the present invention is not deformed in case the bag was dropped, and the bag can be preferably used as a transfer bag of a multiple bag system.

## Claims

1. A bag for storage of platelets made of a resin comprising
a polymer alloy consisting essentially of
(a) 25 to 60 % by weight of poly(ethylene-butylene)-polystyrene block copolymer,
(b) 20 to 40 % by weight of polypropylene, and
(c) 5 to 35 % by weight of ethylene-acrylic acid ester copolymer, the segment of said polystyrene in the molecular chains of said polymer (a) having at least 2 oriented portions and the molecular chains of said polymers (a), (b) and (c) being entangled to form a network structure,
and 1 to 20 % by weight of a plasticizer.

2. The bag for storage of platelets of Claim 1, wherein said plasticizer is at least one member selected from the group of dioctyl phthalate, dioctyl adipate, tri-n-octyl trimellitate and tri-2-ethylhexyl trimellitate.

3. A multiple bag system comprising
(a) a donor bag for receiving blood from a blood donor and separating the components of the blood,
(b) one or more than one transfer bag for storing the separated blood components, and
(c) tubes for connecting a bag with other bag and transferring the separated blood components, wherein at least one transfer bag is made of a resin comprising a polymer alloy consisting essentially of
(i) 25 to 60 % by weight of poly(ethylene-butylene)-polystyrene block copolymer,
(ii) 20 to 40 % by weight of polypropylene, and
(iii) 5 to 35 % by weight of ethylene-acrylic acid ester copolymer, the segment of said polystyrene in the molecular chains of said polymer (i) having at least 2 oriented portions and the molecular chains of said polymers (i), (ii) and (iii) being entangled to form a network structure,
and 1 to 20 % by weight of a plasticizer.

## Patentansprüche

1. Beutel für die Lagerung von Blutplättchen, hergestellt aus einem Harz, das umfaßt
eine Polymerlegierung, bestehend im wesentlichen aus
(a) 25 bis 60 Gew.-% eines Poly(ethylen-butylen)/Polystyrol-Block-Copolymers,
(b) 20 bis 40 Gew.-% Polypropylen und
(c) 5 bis 35 Gew.-% eines Ethylen/Acrylsäureester-Copolymers,
wobei das Segment aus dem Polystyrol in den Molekülketten des Polymers (a) mindestens zwei orientierte Abschnitte aufweist und die Molekülketten der Polymeren (a), (b) und (c) miteinander verfilzt (verhakt) sind unter Bildung einer Netzwerkstruktur, und
1 bis 20 Gew.-% eines Weichmachers.

2. Beutel für die Lagerung von Blutplättchen nach Anspruch 1, wobei der Weichmacher mindestens ein Vertreter ist, der ausgewählt wird aus der Gruppe, die besteht aus Dioctylphthalat, Dioctyladipat, Tri-n-octyl-trimellithat und Tri-2-ethylhexyl-trimellithat.

3. Mehrbeutel-System, das umfaßt
(a) einen Spenderbeutel für die Aufnahme von Blut aus einem Blutspender und die Auftrennung der Blutkomponenten,
(b) einen oder mehr als einen Übertragungsbeutel für die Lagerung der abgetrennten Blut-Komponenten und
(c) Schläuche für das Verbinden eines Beutels mit einem anderen Beutel und die Übertragung der abgetrennten Blutkomponenten, wobei mindestens ein Übertragungsbeutel aus einem Harz hergestellt ist, das umfaßt eine Polymer-Legierung, die im wesentlichen besteht aus
i) 25 bis 60 Gew.-% eines Poly(ethylen-butylen)/Polystyrol-Block-Copolymers,
ii) 20 bis 40 Gew.-% Polypropylen und
iii) 5 bis 35 Gew.-% eines Ethylen/Acrylsäureester-Copolymers,
wobei das Segment aus dem Polystyrol in den Molekülketten des Polymers (i) mindestens zwei orientierte Abschnitte aufweist und die Molekülketten der Polymeren (i), (ii), (iii) miteinander verfilzt (verhakt) sind unter Bildung einer Netzwerkstruktur, und
1 bis 20 Gew.-% eines Weichmachers.

## Revendications

1. Sac pour le magasinage de plaquettes sanguines, fait à base d'une résine comprenant :
un alliage de polymères constitué essentiellement de
(a) 25 à 60% en poids de copolymère du groupe poly(éthylène-butylène)-polystyrène.
(b) 20 à 40% en poids de polypropylène et,
(c) 5 à 35% en poids de copolymère ester acide éthylène-acrylique, le segment dudit polystyrène dans les chaînes moléculaires dudit polymère (a) ayant au moins deux parties orientées et les chaînes moléculaires desdits polymères (a), (b) et (c) étant enchevêtrées pour former une structure réticulée,
et 1 à 20% en poids d'un plastifiant.

2. Sac pour le magasinage de plaquettes sanguines selon la revendication 1, dans lequel ledit plastifiant est au moins un élément choisi à partir du groupe phtalate de dioctyle, adipate de dioctyle, trimellitate de tri-n-octyle et trimellitate de tri-2-éthylehexyle.

3. Système de sacs multiples comprenant
(a) un sac de donneur pour recevoir du sang d'un donneur et séparer les composants du sang,
(b) un ou plus d'un sac de transfert pour emmagasiner les composants du sang séparés et,
(c) des tubes pour raccorder un sac avec un autre sac et transférer les composants du sang séparés, dans lequel au moins un sac de transfert est fait à base d'une résine comprenant un alliage de polymères constitué essentiellement de
(i) 25 à 60% en poids de copolymère du groupe poly(éthylène-butylène)-polystyrène.
(ii) 20 à 40% en poids de polypropylène et,
(iii) 5 à 35% en poids de copolymère ester acide éthylène- acrylique, le segment dudit polystyrène dans les chaînes moléculaires dudit polymère (i) ayant au moins deux parties orientées et les chaînes moléculaires desdits polymères (i), (ii) et (iii) étant enchevêtrées pour former une structure réticulée,
et 1 à 20% en poids d'un plastifiant.
